# EUROPEAN PATENT APPLICATION

(11) **EP 2 929 878 A1**
(43) Date of publication of application: **14.10.2015**
(21) Application number: 15162759.3
(22) Date of filing: 08.04.2015
(51) Int. Cl.: A61K 9/20, A61K 31/195

(54) **EXTENDED RELEASE FORMULATIONS OF GABAPENTIN**

(30) Priority: 09.04.2014 TR 201404106
(71) Applicant: Arven Ilac Sanayi ve Ticaret A.S., Istinye, Istanbul 34460 (TR)
(72) Inventor: Türkyilmaz, Ali, 34460 Istanbul (TR); Erdem, Yelda, 34460 Istanbul (TR); Torun, Hande, 34460 Istanbul (TR)
(74) Representative: Sevinç, Erkan

(57) **Abstract**

The present invention relates to an extended release formulation comprising a therapeutically effective amount of gabapentin and a release retarding agent thereof.

## Description

### Field of Invention

The present invention relates to an extended release formulation comprising a therapeutically effective amount of gabapentin and a release retarding agent thereof.

### Background of Invention

GABA is an inhibitory amino acid found in the mammalian central nervous system (CNS). It has been reported that dysfunctions with GABA neurotransmission in the CNS may contribute or even cause psychiatric and neurological diseases such as epilepsy, schizophrenia, Parkinson's disease, Huntington's chorea and dyskinesia (Saletu B., et al., International Journal of Clinical Pharmacology, Therapy, and Toxicology, 1986; 24: 362-373).

Gabapentin was designed as a GABA analog that would cross the blood-brain barrier. It was found to have anticonvulsant and antispastic activity with extremely low toxicity in man.

Chemical name of gabapentin is 1-(aminomethyl)cyclohexaneacetic acid and it is illustrated with Formula I given below. It is a water soluble and bitter tasting molecule with a structure resembling GABA with cyclohexane ring. It may be exist in three forms as crystalline, hydrate and anhydrous polymorph forms.

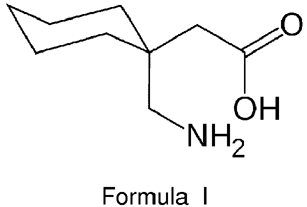

Immediate-release tablets and capsules of Gabapentin are presently marketed under the trademark Neurontin® as adjunctive orally therapy administered three times per day in the strength of 100mg , 300mg, 400mg, 600mg and 800 mg in the treatment of partial seizures in patients with epilepsy and postherpetic neuralgia.
Gabapentin tablet is also commercially available for "once daily" use under the name of GRALISE® in the strengths of 300mg and 600mg. It is taken orally once daily with food (evening meal) and the maximum daily dose of GRALISE® is 1800 mg.

It is a desired feature to obtain an active pharmaceutical agent in an extended release form in the treatment of a number of diseases. The term extended release may be defined as reaching desired plasma levels of an active agent of interest throughout a determined period of time, and providing the drug release at a uniform and constant rate. Thus, the drug administration frequency can be decreased.

There are various difficulties associated with developing an extended release formulation. One of the problems encountered is dose dumping, as a result of too rapid release of the drug. Obtaining intended solubility profiles is also difficult to control the release rate. For this reason, there may occur fluctuations in the plasma concentrations of active agents, which may lead to toxicity. In addition, daily alterations may be possible for the active agent in plasma.

To overcome all these problems and adjust the dissolution profile, during the developing of a drug formulation, a release retarding agent should be used. Retarding agent may be used to form a polymeric matrix or it may be used as a thickening agent. With the help of high viscosity, extended release is achieved.

In addition, gabapentin is an active agent which has a stability problem in the solid state. It has been found to degrade into lactam, resulting in a decrease in the potency of gabapentin over time. Therefore, it is necessary to avoid degradation of gabapentin over the shelf life of the product. Mechanical stress associated with process and using high amount of excipients increase the subsequent lactamization rate upon storage due to increased gabapentin polymorph form disorder. Therefore, it is important to use excipients in a specific amount that decrease the degradation and sustain the stability.

Accordingly, there is a need for an improved extended release formulation of gabapentin or pharmaceutically acceptable salt to achieve desired dissolution profile while overcoming stability and process related problems. Gabapentin has also poor compressibility and compactibility. In this present invention, these problems are also solved by adjusting the ratio of release retarding agent to lubricant.

### Description of the invention

The main embodiment of the present invention is to provide an extended release formulation comprising a therapeutically effective amount of gabapentin and a release retarding agent thereof.

According to one embodiment of the present invention is to obtain an extended release formulation comprising gabapentin for once daily usage with or without food.

In one embodiment, gabapentin is present in an amount of 5 to 90 % by weight of total formulation, preferably it is 25 to 75 % by weight of total formulation and more preferably it is 40 to 75 % by weight of total formulation.

In one embodiment, gabapentin is in the form of anhydrous polymorph form.

As used herein, the term "release retarding agent" is defined as the pharmaceutical ingredient that provides modified release, retarded release, slow release, controlled release, sustained release, prolonged release, zero point order release or extended release.

The term "extended release" may be defined as reaching desired plasma levels of an active agent of interest throughout a determined period of time, and providing the drug release at a uniform and constant rate. Gabapentin is highly soluble in water. High solubility affects the dissolution profile and it may cause dose dumping which is a result of too rapid release of the active agent. In this present invention, to provide an extended release and to control the release rate, release retarding agents have been used. These release retarding agents may be gelling agents which form polymeric matrices or non-gelling agents which are used as a thickening agents.

In one embodiment, release retarding agent is present in an amount of 0.50 - 60.00 %, preferably 5.00 - 50.00% by weight of total formulation.

On the other side, gabapentin has a stability problem in the solid state. It degrades directly to gabapentin-lactam due to high amount of excipients or mechanical stress applied during the process. Lactamization rate upon storage is occured due to increased gabapentin polymorph form disorder. To prevent lactam impurity in the gabapentin formulation and provide superior stability, in this present invention release retarding agent has been used in a specific ratio.

In this embodiment, the ratio of gabapentin to release retarding agent is in the range of 10.00 to 1.00 (w/w), preferably 5.00 to 1.00 (w/w) and more preferably it is 3.00 to 1.00 (w/w).

According to this embodiment, the stability assay has been performed by HPLC at a wavelength of UV 213 nm.

According to one embodiment, the release retarding agent is a non-gelling agent. Non-gelling agents are thickeners that provide extended release by increasing the viscosity of the formulation.

According to this embodiment, the non-gelling agent is selected from the group comprising lambda (λ) carrageenan, ethylcellulose linear (N-type) polymers or copolymers, polymethacrylates or copolymers, natural gums such as xanthan gum, surfactants as cetostearyl alcohol, cethyl alcohol or mixtures thereof. Preferably, it is lambda (λ) carrageenan, ethylcellulose linear (N-type) polymer, polymethacrylate or mixtures thereof.

According to one embodiment, the release retarding agent is a gelling agent. Gelling agents are polymers that form matrices wherein the active agents entrapped. The viscosity of the formulation is increased by gel forming and slow release of active agent is achieved from the polymeric matrix.

According to this embodiment, the gelling agent is selected from the group comprising types of HPMC (hydroxyl propyl methylcellulose) such as HPMC E4M and HPMC K100, hydroxyethyl methylcellulose, hydroxypropyl cellulose, hydroxypropyl ethylcellulose, ethylcellulose, methacrylic acid - ethyl acrylate copolymer , polymethylmetacrylate or copolymers, , polyvinyl acetate, polyvinyl alcohol, polyvinylpyrrolidine, glyceryl behenate, polyethylene oxide, polyethylene glycol, cellulose acetate, vinyl acetate/crotonic acid copolymers, maleic anhydride/methyl vinyl ether copolymers, copolymer of acrylic or methacrylic acid esters, polyoxyethylene -alkyl ethers or mixtures thereof. Preferably it is hydroxyl propyl methylcellulose.

In both cases, the high viscosity around active agent particles slow down the penetration of water into particles and this in turn reduce the dissolution rate of active agent from particles.

According to another embodiment, in- vitro release of gabapentin is measured in 900mL of deionized water at 100 rpm at 37 °C ± 0.5 °C.

Furthermore, gabapentin has compressibility and compactibility problems. Compressibility is the ability of a powder to decrease in volume under pressure, while compactibility is the ability of a powder to be compressed into a tablet of certain hardness or crushing strength.
In prior art, there are some solution about these problems of gabapentin. In contrast to prior art, in this invention, lubricant has been used in a specific ratio of release retarding agent to overcome these problems of gabapentin. It is surprisingly found that the ratio of release retarding agent to lubricant affects the compressibility and compactibility of gabapentin formulation. According to this embodiment, the ratio of release retarding agent to lubricant is between 50.00 to 0.20 (w/w), preferably 25.00 to 0.20 (w/w) and more preferably it is 20.00 to 2.00 (w/w).

In one embodiment, the ratio of release retarding agent to filler is between 10.00 to 0.01 (w/w), preferably 5.00 to 0.10 (w/w) and more preferably it is 3.00 to 0.80 (w/w).

In one embodiment, coating has been preferably used for the formulation of gabapentin due to its bitter taste and moisture sensitivity. In this present invention, preferably, Opadry II Pink has been used as a coating. It is comprised of hypromellose type 2910 USP, titanium dioxide USP, lactose monohydrate NF, polyethylene glycol 3350 NF, triacetin USP, and FD&C Red.

In this present invention, to achieve extended release, superior stability and improved tablet compressibility, these formulations have been designed, comprising the following:
a. 5.00 - 90.00 % by weight of gabapentin
b. 0.50 - 60.00 % by weight of release retarding agent
c. 5.00 - 30.00 % by weight of microcrystalline cellulose
d. 0.10 - 2.50 % by weight of magnesium stearate
e. 0.1 - 2.0 % by weight of colloidal silicon dioxide
f. preferably, coating

Suitable diluents and fillers may include but not limited to microcrystalline cellulose, dibasic calcium phosphate, tribasic calcium phosphate, trehalose, isomalt, mannitol, lactose, starch, sodium carbonate, sodium bicarbonate, calcium carbonate or mixtures thereof, preferably microcrystalline cellulose.

Suitable lubricants may include but not limited to magnesium strearate, sodium stearyl fumarate, stearic acid, calcium stearate or mixtures thereof; preferably magnesium strearate.

Suitable glidants may include but not limited to colloidal silicon dioxide, talc, aluminium silicate or mixtures thereof; preferably talc.

Coating may be selected from the group comprising Polyvinyl alcohol-polyethylene glycol copolymers (Kollicoat IR), polyvinyl alcohol or copolymers or mixtures thereof (Opadry AMB), Ethylcellulose Dispersions (Surelease), Kerry-HPC, polyethylene glycol, polyvinylprolidone, polyvinylprolidone-vinyl acetate copolymer(PVP-VA), and all kinds of OpadryTM such as Opadry pink II, as well as pigments, dyes, titanium dioxide and iron oxide, talk and polymethylmetacrylate copolymers (Eudragit).

### Example 1:

| **ingredients** | **amount (%)** |
|---|---|
| gabapentin | 40.00 - 75.00 |
| HPMC E4M | 5.00 - 25.00 |
| HPMC K100 MCR | 5.00 - 25.00 |
| microcrystalline cellulose PH 101 | 5.00 - 30.00 |
| magnesium stearate | 0.10 - 2.50 |
| colloidal silicn dioxide | 0.10 - 2.00 |
| Opadry II Pink 31F24886 | 1.0 - 15.00 |

| | |
|---|---|
| * HPMC K100 MCR is high viscosity HPMC that has 75.000-140.000 cps grades. HPMC E4M is low viscosity HPMC that has 2.700 - 5.040 cps viscosity grades. | |

The process of the formulation is carried out as follows: gabapentin, microcrystalline cellulose PH 101, HPMC E4M are mixed while sieving. Powder mixture is granulated with ethanol and then, dried. Obtained particles are sieved and colloidal silicon dioxide , magnesium stearate and HPMC K100 MCR are added to mixture and mixed. Total mixture is compressed into tablets and coated with Opadry Pink II.

### Example 2:

| **ingredients** | **amount (%)** |
|---|---|
| gabapentin | 40.0 - 75.00 |
| ethylcellulose (N Type) | 2.00 - 25.00 |
| microcrystalline cellulose PH 101 | 5.00 - 30.00 |
| magnesium stearate | 0.10 - 2.50 |
| colloidal silicn dioxide | 0.10 - 2.00 |
| Opadry II Pink 31F24886 | 1.0 - 15.00 |

The process of the formulation is carried out as follows: The process of the formulation is carried out as follows: gabapentin, microcrystalline cellulose PH 101, ethylcellulose are mixed while sieving. Powder mixture is granulated with deionized water and then, dried. Obtained particles are sieved and colloidal silicon dioxide and magnesium stearate are added to mixture and mixed. Total mixture is compressed into tablets and coated with Opadry Pink II.

### Example 3:

| **ingredients** | **amount (%)** |
|---|---|
| gabapentin | 40.0 - 75.00 |
| lambda Carragenan | 0.50 - 10.00 |
| microcrystalline cellulose PH 101 | 5.00 - 30.00 |
| magnesium stearate | 0.10 - 2.50 |
| colloidal silicn dioxide | 0.10 - 2.00 |
| Opadry II Pink 31F24886 | 1.0 - 15.00 |

The process of the formulation is carried out as follows: The process of the formulation is carried out as follows: gabapentin, microcrystalline cellulose PH 101, lambda carragenan are mixed while sieving. Powder mixture is granulated with deionized water and then, dried. Obtained particles are sieved and colloidal silicon dioxide , magnesium stearate and are added to mixture and mixed. Total mixture is compressed into tablets and coated with Opadry Pink II.

### Example 4:

| **ingredients** | **amount (%)** |
|---|---|
| gabapentin | 40.0 - 75.00 |
| polymethacrylate (PMA) | 5.00 - 50.00 |
| microcrystalline cellulose PH 101 | 5.00 - 30.00 |
| magnesium stearate | 0.10 - 2.50 |
| colloidal silicn dioxide | 0.10 - 2.00 |
| Opadry II Pink 31F24886 | 1.0 - 15.00 |

The process of the formulation is carried out as follows: The process of the formulation is carried out as follows: gabapentin, microcrystalline cellulose PH 101 polymethacrylate are mixed while sieving. Powder mixture is granulated with ethanol water mixture and then, dried. Obtained particles are sieved and colloidal silicon dioxide and magnesium stearate are added to mixture and mixed. Total mixture is compressed into tablets and coated with Opadry Pink II.

## Claims

1. An extended release formulation comprising a therapeutically effective amount of gabapentin and a release retarding agent thereof.

2. The extended release formulation according to claim 1, wherein gabapentin is present in an amount of 5 to 90 % by weight of total formulation, preferably it is 25 to 75 % by weight of total formulation and more preferably it is 40 to 75 % by weight of total formulation.

3. The extended release formulation according to claim 1, wherein gabapentin is in the form of anhydrous polymorph form.

4. The extended release formulation according to claim 1, wherein the ratio of gabapentin to release retarding agent is in the range of 10.00 to 1.00 (w/w), preferably 5.00 to 1.00 and more preferably it is 3.00 to 1.00 (w/w).

5. The extended release formulation according to claim 1, wherein the release retarding agent is a non-gelling agent or a gelling agent.

6. The extended release formulation according to claim 5, wherein the non-gelling agent is selected from the group comprising lambda (λ) carrageenan, ethylcellulose linear (N-type) polymers or copolymers, polymethacrylates or copolymers, natural gums such as xanthan gum, surfactants as cetostearyl alcohol, cethyl alcohol or mixtures thereof, preferably, it is lambda (A) carrageenan, ethylcellulose linear (N-type) polymer, polymethacrylate or mixtures thereof.

7. The extended release formulation according to claim 5, wherein the gelling agent is selected from the group comprising types of HPMC (hydroxyl propyl methylcellulose), hydroxyethyl methylcellulose, hydroxypropyl cellulose, hydroxypropyl ethylcellulose, ethylcellulose, methacrylic acid - ethyl acrylate copolymer, polymethylmetacrylate or copolymers, , polyvinyl acetate, polyvinyl alcohol, polyvinylpyrrolidine, glyceryl behenate, polyethylene oxide, polyethylene glycol, cellulose acetate, vinyl acetate/crotonic acid copolymers, maleic anhydride/methyl vinyl ether copolymers, copolymer of acrylic or
methacrylic acid esters, polyoxyethylene -alkyl ethers or mixtures thereof, preferably it is hydroxyl propyl methylcellulose.

8. The extended release formulation according to any preceding claims, wherein the ratio of release retarding agent to lubricant is between 50.00 to 0.20 (w/w), preferably 25.00 to 0.20 (w/w) and more preferably it is 20.00 to 2.00 (w/w).

9. The extended release formulation according to any preceding claims comprising;
a. 5.00 - 90.00 % by weight of gabapentin
b. 0.50 - 60.00 % by weight of release retarding agent
c. 5.00 - 30.00 % by weight of microcrystalline cellulose
d. 0.10 - 2.50 % by weight of magnesium stearate
e. 0.1 - 2.0 % by weight of colloidal silicon dioxide
f. preferably, coating

10. The extended release formulation according to any preceding claims comprising;
a. 40.00 - 75.00 % by weight of gabapentin
b. 5.00 - 25.00 % by weight of HPMC (low viscosity)
c. 5.00 - 25.00 % by weight of HPMC (high viscosity)
d. 5.00 - 30.00 % by weight of microcrystalline cellulose
e. 0.10 - 2.50 % by weight of magnesium stearate
f. 0.1 - 2.0 % by weight of colloidal silicon dioxide
g. preferably, coating

11. The extended release formulation according to any preceding claims comprising;
a. 40.00 - 75.00 % by weight of gabapentin
b. 2.00 - 25.00 % by weight of ethylcellulose (N Type)
c. 5.00 - 30.00 % by weight of microcrystalline cellulose
d. 0.10 - 2.50 % by weight of magnesium stearate
e. 0.1 - 2.0 % by weight of colloidal silicon dioxide
f. preferably, coating

12. The extended release formulation according to any preceding claims comprising;
a. 40.00 - 75.00 % by weight of gabapentin
b. 0.50 - 10.00 % by weight of lambda carragenan
c. 5.00 - 30.00 % by weight of microcrystalline cellulose
d. 0.10 - 2.50 % by weight of magnesium stearate
e. 0.1 - 2.0 % by weight of colloidal silicon dioxide
f. preferably, coating

13. The extended release formulation according to claim 10 comprising;
a. 40.00 - 75.00 % by weight of gabapentin
b. 5.00 - 50.00 % by weight of polymethacrylate (PMA)
c. 5.00 - 30.00 % by weight of microcrystalline cellulose
d. 0.10 - 2.50 % by weight of magnesium stearate
e. 0.1 - 2.0 % by weight of colloidal silicon dioxide
f. preferably, coating
